# EUROPEAN PATENT SPECIFICATION

(11) **EP 0 553 925 B1**
(45) Date of publication and mention of the grant of the patent: **08.04.1998**
(21) Application number: 93200191.0
(22) Date of filing: 26.01.1993
(51) Int. Cl.: C07D 487/22, C07D 519/00, A61K 31/40

(54) **Porphyrin derivatives for diagnosis and photodynamic therapy**
Phorphyrinderivate für Diagnose und photodynamischer Therapie
Dérivés de la porphyrine pour diagnose et thérapie photodynamique

(30) Priority: 27.01.1992 NL 9200143
(43) Date of publication of application: 04.08.1993
(73) Proprietor: SEEHOF LABORATORIUM F&E GESELLSCHAFT, D-25764 Wesselburener Koog (DE)
(72) Inventor: Mueller von der Haegen, Hans, W-2244 Wesselburenerkoog (DE)
(74) Representative: Van kan, Johan Joseph Hubert, Ir.

(56) References cited:
- WO-A-84/01382

## Description

The invention relates to a process for the preparation of an active compound, to be used for photodynamic therapy and for making a diagnosis, by starting from a porphyrin compound which porphyrin compound is dissolved in an aqueous solution and reacted at a substantially neutral pH-value to form an oligomer product. Furthermore, the invention relates to the active compound that can be used as a therapeutic substance and as a diagnostic substance, as well as to the pharmaceutic composition containing said active compound. The starting material for the reaction is a porphyrin compound that can de derived from blood, although such compounds can also be obtained by technical or biotechnical synthesis.

This process is known from US-A-4,882,234. Acccording to this patent an ester-linked oligomeric porphin-based material is prepared having an activity as a photoimaging and photodynamic therapy agent. However the thus obtained substance used as a therapeutic substance still has side-effects which should be avoided. This avoidance of side-effects is possible according to the invention.

From EP-A-120.054 a compound is known which can be used as a diagnostic substance and as a substance for treating tumors, said compound being fluorescent and photosensitive, by starting from hematoporphyrin, which is reacted with acetic acid/sulphuric acid, the resulting derivative is precipitated, the pH of the obtained aqueous suspension of the derivative is adjusted to 7 - 7.4, after which the high molecular weight derivative is separated by filtration by using a membrane, so as to separate substances with a molecular weight below 10,000. Porphyrin compounds are derivatives of porphin.

The photodynamic therapy of cancer patients is based on a porphyrin derivative being intravenously administered to patients, who are then irradiated with visible light, so that under the influence of the light the porphyrin derivative will selectively attack the malignant cells. The present pharmaceutical composition can also be used against several other diseases as plague, papillomatosis, psoriasis and warts. The therapeutic effect is based on the fact that irradiation of the cells in which the therapeutic drug is present will cause damage to said tumor cells. This therapy is described by Diamond et al. in "Photodynamic Therapy of Malignant Tumors", Lancet 2, 1175-1177, 1973, by Dougherty et al. in "Photoradiation Therapy for the Treatment of Malignant Tumors", Cancer Res. 38, 2628-2635, 1978, and in US-A-4,866,168 (1989), which partially corresponds to the above-mentioned European Patent No. 120,054.

The therapeutic drugs that have been prepared in accordance with said European Patent No. 120,054 have side effects, whilst their preparation is rather difficult because of the comparatively difficult filtration through a membrane system.

These drawbacks have now been overcome by using a method according to the invention, which is characterized in that the reaction is continued until a substantially constant value for the fluorescence with ultraviolet light is obtained, whereupon from the obtained reaction mixture a substantially with ultraviolet light non-fluorescent first compound is separated, said first compound being processed into the substance to be used therapeutically, whilst the fluorescent compound can be used as a diagnostic substance.

After the reaction the oligomer is separated by normal filtration or by extraction, so that a diagnostic substance (fluorescent compound) and a therapeutic substance (non-fluorescent compound) are obtained. This separation makes it possible to obtain a therapeutic substance of which the side effects are minimized, because it is possible to substantially reduce the period following therapy of no exposure to light for the patients. The diagnostic substance is administered in considerably smaller doses than those for the substance for therapeutic purposes (for diagnostic purposes only 20% of the drug required for therapeutic purposes is used), resulting in a considerable enhancement of the tolerance of therapeutic substance. The process according to the invention provides a final product of porphyrin compounds about 20% of which consists of a fluorescent compound (diagnostic substance), whilst about 80% of the final product consists of a non-fluorescent compound (therapeutic substance). Following the liquid extraction the two compounds are separated from each other with a purity of about 99%.

By using the method according to the invention a porphyrin derivative is obtained which is non-fluorescent and which provides a potent selective action in tumors because of its photosensitive effect. This method utilizes alkali salts and alkaline earth salts of hematoporphyrin compounds, protoporphyrin compounds, intermediate products thereof, acyl compounds and derivatives, as well as mixtures of the said compounds as starting materials.

The starting materials are mixed in an aqueous solution and following dissolution the mixture is adjusted to a value in the range of pH = 6.8. While possibly increasing the temperature the starting materials are then reacted and the progress of the reaction can be monitored by measuring the absorption spectrum in the range of 402 nm. The pH of a sample taken is adjusted, after which an absorption spectrum is recorded with light of about 402 nm. As soon as the spectrum no longer changes or approximates a certain limiting value, the reaction can be considered as terminated. Dependent on the composition, the concentration of the starting materials, the reaction temperature and the pH-value of the solution, the end of the reaction is reached within a period of 15 minutes to 100 hours.

Another indication for the progress of the reaction is the change in the intensity of the fluorescence upon irradiation with ultraviolet light. Contrary to the starting materials, the new substance, obtained in accordance with the invention which is non-fluorescent and which can be used as a therapeutic substance, does not exhibit any fluorescence. Consequently, the reaction is considered as terminated when the fluorescence of the solution no longer decreases.

As indicated above the mixture of porphyrin derivatives obtained in accordance with this reaction consists of a fluorescent compound for diagnostic purposes and of a non-fluorescent compound, which can be used for therapeutic purposes. In accordance with the invention this mixture of compounds can be separated by using extraction with a partly or totally water miscible organic solvent, whereby the fluorescent compound is obtained as a solution and the non-fluorescent compound is left as a solid. The solid can then be recovered by various methods, such as filtration, centrifugation, absorption and the like. The separation of the non-fluorescent solid can be determined from the complete disappearance of the fluorescence of the obtained compound. The determination of the fluorescence is in particular carried out in an aqueous, acid solution with a measurement of the absorption with 402 nm light. Furthermore, the therapeutic substance according to the invention can be characterized by the value of the extinction coefficient in an aqueous acid solution when the extinction is measured at 365 nm and at 402 nm, whereby the ratio reaches a value of 1.3 ± 5% being about 0.05. This is further illustrated in the following description, in which reference is made to the appended drawings, in which:

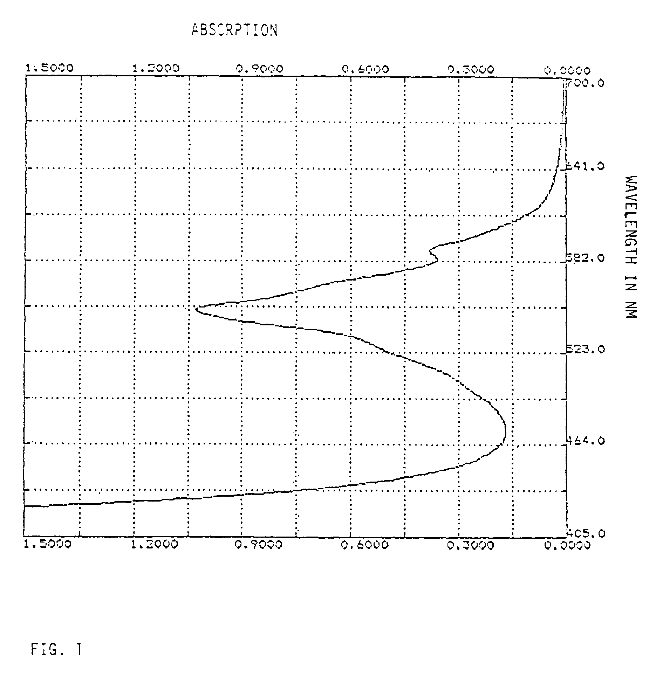

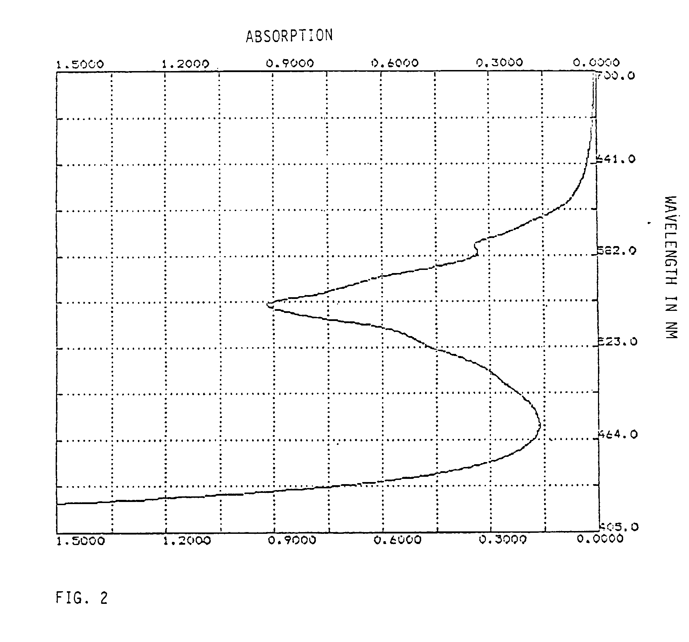

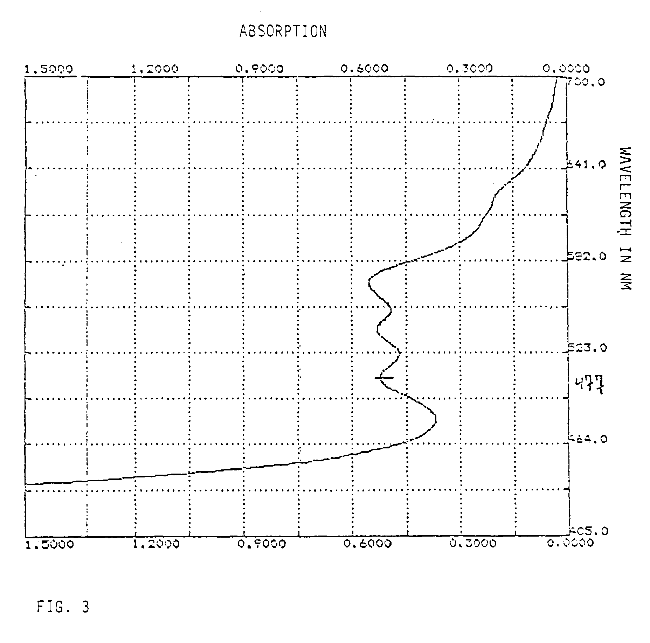

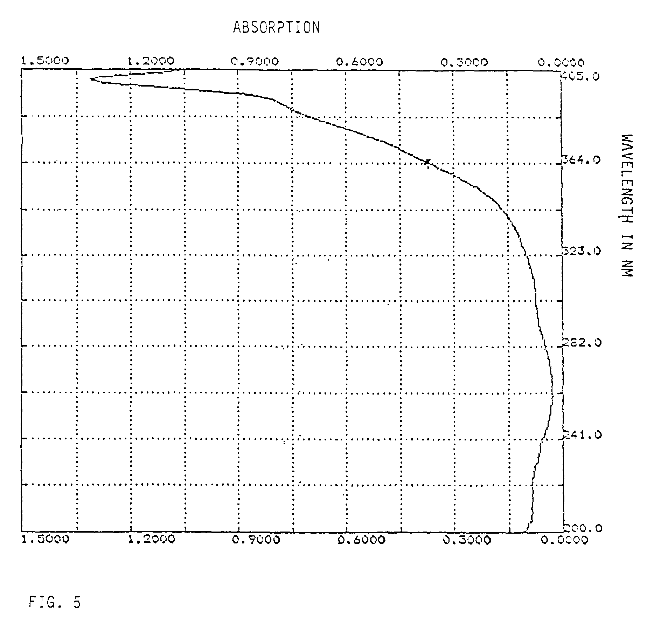

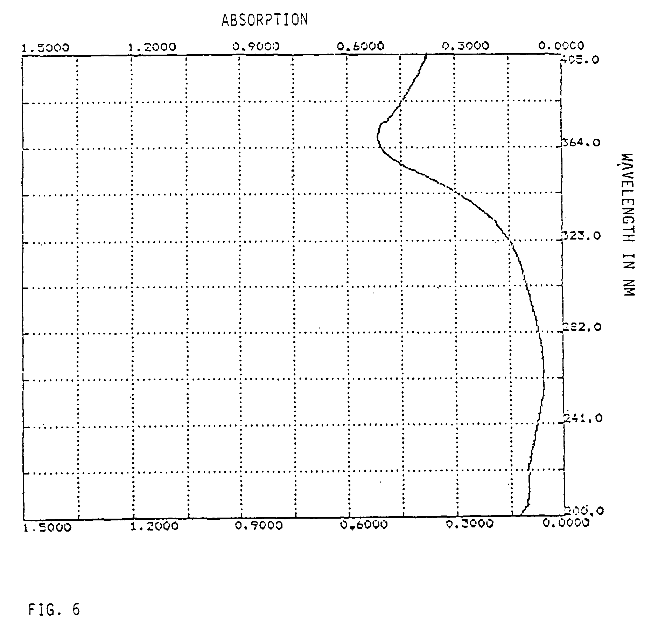

Figures 1 - 6 show UV-absorption spectra of various porphyrin-containing compounds, recorded between 200 and 700 nm.

In order to improve the therapeutical use of the obtained solid, said solution thereof may be mixed with sugars, such as mannitol, glucose, and subsequently be solidified by lyophilization. For systemic application, the compound is preferably dissolved in water, the pH of the solution is adjusted to 7.4 and the solution is made isotonic.

The invention will be explained in more detail with reference to the following examples of the preparation of the therapeutic substance and the diagnostic substance according to the invention.

### Example I

Using a method known as such porphyrin or porphyrin derivatives are prepared from blood or blood preparation, and used as starting materials for the preparation of the therapeutic substance and the diagnostic substance according to the invention.

For this purpose 600 g of hematoporphyrin base (commercially available from Harimex B.V., Loenen, NL) or 627 g of hematoporphyrin dimethyl ester (commercially available from Harimex B.V., Loenen, NL) (or mixtures thereof) is slowly mixed, while stirring, with 10 l of 0.2 N sodium hydroxide, at a temperature of 20 °C. After the starting materials is completely dissolved, the pH of the solution is adjusted to a value of 7.5 using 1 N hydrochloric acid, and the stirring is continued. The progress of the reaction is followed by taking samples from the fluid and measuring the fluorescence value thereof with ultraviolet light. The reaction rate can be increased by raising the temperature of the solution. The reaction can be considered complete when the intensity of the fluorescence no longer decreases.

After the fluorescence value has become constant, the further purification of the fluorescent and the non-fluorescent compounds is carried out by extraction of the fluorescent compound with 2-propanol. A solid containing the therapeutic compound is left. The extraction is complete when the fluorescent compound has been completely removed. The completion of the purificiation process can be deduced from the absence of fluorescence of the product in an aqueous solution when said solution is irradiated with ultraviolet light.

### Example II

As a starting material 6 g of hematoporphyrin base as indicated in Example I is used and this starting material is dissolved in 100 ml 0,2 N NaOH in water. The pH of the solution is adjusted to a value of 7.5 with 1 N HCl.

Half of this solution is maintained at an elevated pressure at a temperature of 120 °C for 6 hours and subsequently at ambient conditions for 94 hours. The other half is maintained at ambient conditions for 100 hours.

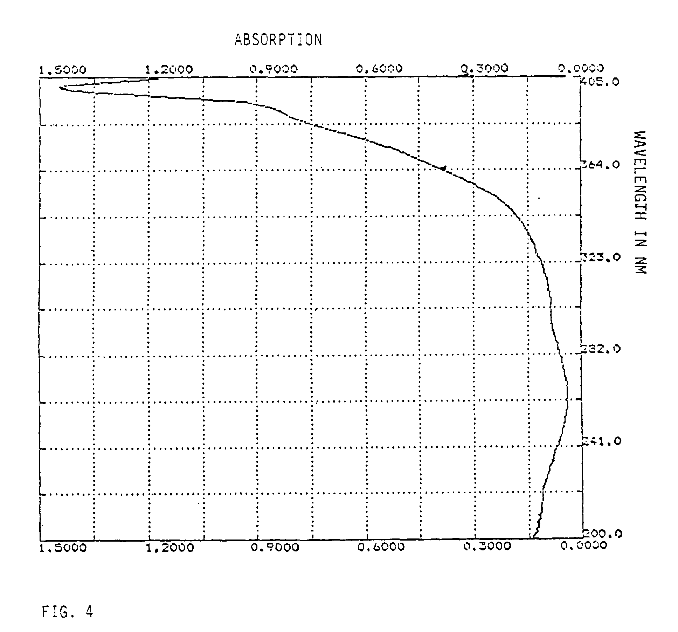

After dilution with methanol/water/phosphoric acid (1000/100/4.75 parts by weight) spectra are recorded, using a Beckman DU-40 spectrophoto-meter, wherby the spectra recorded between 700 and 405 nm are shown in Figures 1 - 3 and those recorded between 405 and 200 nm are shown in Figures 4 - 6. Table A shows the absorption peaks taken at 700 - 405 nm, with a dilution factor of 1000, and at 405 - 200 nm, with a dilution factor of 15000.

**TABLE A**

| absorption peaks at 700 - 200 nm | | |
|---|---|---|
| sample | absorption peaks in nm | |
| | dilution 1000 x | dilution 15,000 x |
| blank | 589 550 | 401 |
| 100 hours at room temperature | 588 550 | 401 |
| 6 hours at 120 °C | 568 538 477 | 368 |

As a blank 6 g of hematoporphyrin base was dissolved in 0.2 N NaOH and the spectrum was recorded.

Table A shows that the spectra of the blank and those of the mixture that was maintained at room temperature for 100 hours are equal, whereas the spectrum of the mixture that was maintained at a tempeature of 120 °C strongly deviates therefrom. Actually, the reaction takes place in a relatively short time, at an elevated temperature.

Table B shows the ratio of the absorptions of the therapeutic substance, measured at 365 nm (A1) and at 401 nm (A2).

**TABLE B**

| ratio of the absorptions of the therapeutic substance | | | |
|---|---|---|---|
| sample | 365 nm A1 | 402 nm A2 | A1/A2 |
| blank | 0.370 | 1.445 | 0.256 |
| 100 hours at room temperature | 0.380 | 1.311 | 0.290 |
| 6 hours at 120 °C | 0.514 | 0.379 | 1.356 |

From this table it can be derived that following the reaction the coefficient reaches a value of 1.356.

Furthermore an analysis based on high-pressure liquid chromatography was carried out, also on the basis of this analysis it appeared that the reaction had taken place at an elevated temperature, whereas measurement of the blank and of the mixture that had been maintained at ambient conditions for 100 hours practically resulted in the same chromatogram.

This example shows that the reaction takes place at a reasonable rate at an elevated temperature and that it can only very slowly take place at ambient conditions.

## Claims

1. Process for the preparation of an active compound, to be used for photodynamic therapy and for making a diagnosis, by starting from a porphyrin compound which porphyrin compound is dissolved in an aqueous solution and reacted at a substantially neutral pH-value to form an oligomer product, characterized in that said reaction is continued until a substantially constant value for the fluorescence with ultraviolet light is obtained, whereupon from the obtained reaction mixture a substantially with ultraviolet light non-fluorescent first compound is separated, said first compound being processed into the substance to be used therapeutically, whilst the remaining fluorescent second compound can be used as a diagnostic substance.

2. Process according to claim 1, characterized in that the first non-fluorescent compound being a solid material is separated from the reaction mixture.

3. Process according to claim 1, characterized in that the non-fluorescent first compound is extracted from the reaction mixture with a partly or totally water miscible organic solvent.

4. Process according to claim 3, characterized in that the extraction is carried out with 2-propanol.

5. A porphyrin based therapeutic substance, characterized in that in an acidic aqueous solution the ratio of the extinction values of said substance at 365 nm and 402 nm is 1.3 ± 5%.

6. A therapeutic substance containing the therapeutically active compound, obtained in accordance with the process of claims 1 - 4.

7. A diagnostic substance containing the diagnostically active compound, prepared in accordance with the process of claims 1 - 4.

8. Pharmaceutical composition containing the therapeutic substance as disclosed in claim 6.

## Patentansprüche

1. Verfahren zum Zubereiten einer aktiven Verbindung, die zur photodynamischen Therapie und zur Diagnose verwendet werden soll, ausgehend von einer Porphyrinverbindung, die in einer wäßrigen Lösung gelöst und bei einem im wesentlichen neutralen pH-Wert zur Reaktion gebracht wird, um ein Oligomerprodukt zu bilden, dadurch gekennzeichnet, daß die Reaktion fortgesetzt wird, bis ein im wesentlichen konstanter Wert für die Fluoreszenz mit Ultraviolettlicht erzielt wird, woraufhin von dem erhaltenen Reaktionsgemisch eine im wesentlichen mit Ultraviolettlicht nicht fluoreszierende erste Verbindung abgetrennt wird, die in eine Substanz verarbeitet wird, die therapeutisch eingesetzt werden soll, während die verbleibende fluoreszierende zweite Verbindung als Diagnosesubstanz eingesetzt werden kann.

2. Verfahren nach Anspruch 1, dadurch gekennzeichnet, daß die erste nicht-fluoreszierende Verbindung ein Festkörpermaterial ist, das von der Reaktionsmischung abgetrennt wird.

3. Verfahren nach Anspruch 1, dadurch gekennzeichnet, daß die nicht-fluoreszierende erste Verbindung von dem Reaktionsgemisch mit einem teilweise oder vollständig wassermischbaren organischen Lösungsmittel extrahiert wird.

4. Verfahren nach Anspruch 3, dadurch gekennzeichnet, daß die Extraktion mit 2-Propanol ausgeführt wird.

5. Auf Porphyrin basierende therapeutische Substanz, dadurch gekennzeichnet, daß in wäßriger Säurelösung das Verhältnis der Extinktionswerte der Substanz bei 365 nm und 402 nm 1,3 ± 5% beträgt.

6. Therapeutische Substanz, enthaltend die therapeutisch aktive Verbindung, die in Übereinstimmung mit dem Verfahren nach Anspruch 1 bis 4 erhalten wird.

7. Diagnosesubstanz, enthaltend die diagnostisch aktive Verbindung, die in Übereinstimmung mit dem Verfahren nach Anspruch 1 bis 4 zubereitet ist.

8. Pharmazeutische Zusammensetzung, enthaltend die therapeutische Substanz, die in Anspruch 6 offenbart ist.

## Revendications

1. Procédé pour la préparation d'un composé actif à utiliser en thérapie photodynamique et pour réaliser un diagnostic, en partant d'un composé de porphyrine, lequel composé de porphyrine est dissous dans une solution aqueuse et mis à réagir à un pH d'une valeur essentiellement neutre pour former un produit oligomère, caractérisé en ce que ladite réaction est poursuivie jusqu'à l'obtention d'une valeur essentiellement constante de la fluorescence en lumière ultraviolette, suite à quoi on sépare du mélange de réaction ainsi obtenu un premier composé essentiellement non fluorescent en lumière ultraviolette, ledit premier composé étant transformé en la substance à utilisation thérapeutique, tandis que le deuxième composé, fluorescent, peut être utilisé comme substance de diagnostic.

2. Procédé selon la revendication 1, caractérisé en ce que le premier composé, non fluorescent, qui se présente sous la forme d'un produit solide, est séparé du mélange de réaction.

3. Procédé selon la revendication 1, caractérisé en ce que le premier composé, non fluorescent, est extrait du mélange de réaction à l'aide d'un solvant organique partiellement ou totalement miscible avec l'eau.

4. Procédé selon la revendication 3, caractérisé en ce que l'extraction est effectuée à l'aide de 2-propanol.

5. Substance thérapeutique à base de porphyrine, caractérisée en ce que dans une solution aqueuse acide, le rapport entre les valeurs d'extinction de ladite substance à 365 nm et à 402 nm est de 1,3 ± 5 %.

6. Substance thérapeutique contenant le composé thérapeutiquement actif obtenu selon le procédé des revendications 1 à 4.

7. Substance de diagnostic contenant le composé actif de diagnostic préparé selon le procédé des revendications 1 à 4.

8. Composition pharmaceutique contenant la substance thérapeutique selon la revendication 6.
